# EUROPEAN PATENT APPLICATION

(11) **EP 3 187 176 A1**
(43) Date of publication of application: **05.07.2017**
(21) Application number: 16207100.5
(22) Date of filing: 28.12.2016
(51) Int. Cl.: A61K 9/20, A61K 9/28, A61K 9/48, A61K 31/519

(54) **PALIPERIDONE MINI TABLETS**

(30) Priority: 29.12.2015 TR 201517253
(71) Applicant: Sanovel Ilac Sanayi ve Ticaret A.S., 34460 Istanbul (TR)
(72) Inventor: TÜRKYILMAZ, Ali, 34460 Istanbul (TR); ZENGINER, Sibel, 34460 Istanbul (TR); YAG, Göksu, 34460 Istanbul (TR)
(74) Representative: Sevinç, Erkan

(57) **Abstract**

The present invention is related to an extended release pharmaceutical composition for oral administration comprising; a mini tablet core comprising one or more water-soluble polymers, an inner layer comprising paliperidone or a pharmaceutically acceptable salt thereof and a coating layer.

## Description

The present invention is related to an extended release pharmaceutical composition for oral administration comprising; a mini tablet core comprising one or more water-soluble polymers, an inner layer comprising paliperidone or a pharmaceutically acceptable salt thereof and a coating layer.

### Background of the Invention

Paliperidone corresponding to the compound 4H-Pyrido[1,2-a]pyrimidin-4-one,3-[2-[4- (6-fluoro-1,2-benzisoxazol-3-yl)-1- piperidinyl]ethyl]-6,7,8,9-tetrahydro-9- hydroxy-2-methyl is an antipsychotic agent which is approved for the treatment of schizophrenia including acute treatment and recurrence prevention. Its chemical structure is shown in the Formula 1. Paliperidone, first described in US5254556A, is available as Invega® Extended Release Tablet and as Xeplion® as sustained release suspension for injection. 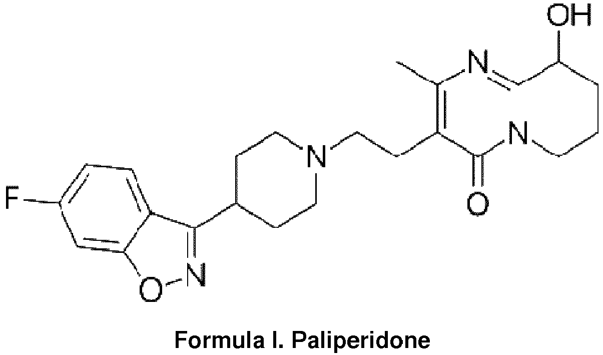 Besides, many solid oral dosage forms are disclosed in prior art. EP2079446 is about a tablet comprising a first layer of paliperidone with at least one release controlling polymer and an outer layer coating partially this first layer. Furthermore, EP2326312 discloses solid pharmaceutical composition comprising at least one solid matrix particle of paliperidone which is a monolithic matrix system. Furthermore, the patent US5254556A discloses oral drops, oral solution, capsules, film-coated tablets, injectable solution and suppositories.

In comparison to prior art, mini tablets have many advantages. It combines the advantages of pellets with the easy process of tableting. Because of their smooth surface, low degree of porosity and high mechanical strength, the mini tablets are easier to give a highly stable film coating which have an uneven surface and high porosity.

Acoording to prior art, extended release mini tablet comprising paliperidone hasn't been attained yet. In this invention, mini tablet formulation for paliperidone has been developed with a desired dissolution profile.

### Detailed Description of the Invention

The main object of the present invention is to provide stabilized extended release pharmaceutical composition for oral administration comprising paliperidone or a pharmaceutically acceptable salt thereof without incorporating surfactant and/or water penetration enhancer.

Another object of the invention is to provide such stabilized extended release pharmaceutical composition for oral administration that exhibit desired in vitro dissolution profile of paliperidone or a pharmaceutically acceptable salt thereof with lag period.

In this invention, an extended release pharmaceutical composition for oral administration comprising;
- a mini tablet core comprising one or more water-soluble polymers,
- an inner layer comprising paliperidone or a pharmaceutically acceptable salt thereof,
- a coating layer.

According to this invention, the extended release pharmaceutical composition for oral administration, wherein the dosage form is in the form of a capsule.

The term "mini tablet", as used herein, refers to small tablets with a diameter equal to or less than 4 mm that are typically filled into a capsule or further compressed into larger tablets. Thickness of this mini tablets equal to or less than 3 mm. The mini tablets have round shape and smooth surface to ease coating process.

The term "dosage form", as used herein, refers to a compact composition comprising three drug layers and one of them is a push layer which is a mini tablet in the core. The compact composition of the present invention has a defined shape such as tablet, capsule, and the like. In particular, it is capsule shaped.

The term "paliperidone" as used herein refers to paliperidone as well as pharmaceutically acceptable salts, enantiomers, hydrates, solvates, metabolites, prodrugs and mixture thereof. The term also includes all polymorphic forms, whether crystalline or amorphous.

According to an embodiment of the present invention, the dosage form comprises paliperidone is present as paliperidone base is in an amount between 1.5 -12 mg.

According to an embodiment of the present invention, the dosage form comprises paliperidone is present as paliperidone base is preferably in an amount of 1.5 mg, 3 mg, 6 mg, 9 mg and 12 mg.

According to an embodiment of the present invention, the extended release pharmaceutical composition comprises paliperidone in an amount of 1.5% to 2.5% of total weight of the pharmaceutical composition, more preferably 1.7% to 2.3% of total weight of the pharmaceutical composition.

According to another embodiment of the present invention, the inner layer comprises paliperidone in an amount of 5% to 30% of weight of the inner layer, more preferably 8% to 25% of total weight of the inner layer.

Mini tablets have many advantages. Some benefits of mini-tablets include excellent size uniformity, regular shape and a smooth surface. In mini tablets, smooth surface offers an excellent substrate for coating with polymeric systems. Therefore, in this invention, a smooth and strong coating which provides high stability mechanically and chemically has been achieved for paliperidone. In addition, mini tablets provides ease for dosing to produce dosage forms comprising paliperidone is present as paliperidone base is in an amount between 1.5 - 12 mg. For dosage form comprising 1.5 mg paliperidone, 2 mini tablets are used for every capsule. For dosage form comprising 3 mg paliperidone, 4 mini tablets are used for every capsule. For dosage form comprising 6 mg paliperidone, 8 mini tablets are used for every capsule. For dosage form comprising 9 mg paliperidone, 12 mini tablets are used for every capsule. For dosage form comprising 12 mg paliperidone, 18 mini tablets are used for every capsule. The superior effect of the mini tablets in this present invention is different amounts of dosage form can be manufactured in the same production line.

The extended release pharmaceutical composition for oral administration comprises at least one pharmaceutically acceptable excipient which is selected from water-soluble polymers, osmotic agents, lubricants, coating materials or mixtures thereof.

Suitable water-soluble polymer is selected from hydroxypropyl cellulose (HPC), hydroxypropyl methylcellulose (HPMC), hydroxypropyl methylcellulose E3, E5 and E15 and K-3, hydroxyethylcellulose and derivatives, ethylcellulose and ethyl hydroxyethylcellulose, carboxymethylcellulose (CMC), carboxymethylcellulose cekol 30, methyl cellulose (MC), methyl cellulose A-3, A6 and A-15; polyvinyl alcohol (PVA), polyethylene glycol (PEG), polyethylene oxide (PEO), poly(ethylene oxide-b-propylene oxide), polyoxyethylene (POE), poly vinyl pyrrolidone (PVP), polyethylenimine (PEI), poly(N-vinylpyrrolidone/vinyl acetate), polyvinylpyrollidone PVP K-90, polyacrylic acid and copolymers, poly(vinylamine) hydrochloride, poly(acrylic acid sodium salt), poly(methacrylic acid), poly(methylacrylic acid sodium salt), poly(ethylene/acrylic acid), poly(2-hydroxyethyl methacrylate/methacrylic acid), poly(2-hydroxypropyl methacrylate), polyacrylamides (PAM), poly(acrylamide/acrylic acid), polymethacrylamide, poly(N-isopropylacrylamide) (PNIPAM), poly(styrenesulfonic acid) sodium salt, poly(2-oxazoline), poly(2-ethyl-2-oxazoline), poly(3-chloro-2-hydroxypropyl-2-methacryloxyethyldimethylammonium chloride), poly(2-vinyl-1-methylpyridinium bromide), poly(2-vinylpyridine), polyamines, poly(2-vinylpyridine N-oxide), poly(N-vinylpyrrolidone/2-dimethylaminoethyl methacrylate) dimethyl sulfate quaternary, poly(4-vinylpyridine N-oxide), poly(4-vinylpyridine), poly(styrenesulfonic acid/maleic acid) sodium salt, poly(N-vinyl acetamide), poly(N-vinyl acetamide-co-sodium acrylate), poly(vinylsulfonic acid) sodium salt, polyelectrolytes; agar, alginates, casein and caseinates, carrageenan, chitosan, cucurbit[n]uril hydrate, curdlan, dextran, gelatin, gellan gum, guar gum and derivatives, gum carrageenan, gum arabic and other tree and shrub exudates, locust bean gum, maltodextrins, methocel, pectin, pullulan, resin, poly(ethylene oxide), poly(ethylene oxide) resin, sodium alginate, starch and starch derivatives, xanthan gum or mixture thereof.

In a preferred embodiment, the mini tablet core of the present invention comprises water-soluble polymer in an amount of more than about 50% w/w, and in particular more than about 70% w/w of the total weight of the mini tablet core.

In another preferred embodiment, the mini tablet core of the present invention comprises water-soluble polymer in an amount of more than about 40% w/w, and in particular more than about 55% w/w of the total weight of the pharmaceutical composition.

In another preferred embodiment, the weight ratio of water-soluble polymer to paliperidone is 15 to 30.

In a preferred embodiment, the water-soluble polymer is preferaly polyethylene oxide.

The term "polyethylene oxide", as used herein, is a non-ionic homopolymer of the formula -(-0-CH2-CH2-)n-, wherein n represents the average number of oxyethylene groups, n generally being from about 2,000 to about 100,000 to 200,000 daltons. It is a water- soluble resin which is available as a white powder in several grades having different molecular weights which vary in viscosity profile when dissolved in water (National Formulary XVII, pp. 1963-1964 (1990)), Polyethylene oxide resin is commercially available under the trade name Polyox®. It is available in various grades depending on its molecular weight, which may range from about 100,000 to about 7,000,000, including from about 200,000 to about 5,000,000 daltons. Examples of suitable grades of polyethylene oxide that may be used in the present invention include Polyox , Polyox WSRN-80 (molecular weight of 200,000 daltons), Polyox® WSR N-750 (molecular weight of 300,000), Polyox® WSR-205 (molecular weight of 600,000 daltons), Polyox® WSR- 1105 (molecular weight of 900,000 daltons), Polyox® WSR N-12K (molecular weight of 1,000,000 daltons), Polyox® WSR N- 60K (molecular weight of 2,000,000 daltons), Polyox® WSR-301 (molecular weight of 4,000,000 daltons), Polyox® WSR Coagulant (molecular weight of 5,000,000 daltons).

In a preferred embodiment, the mini tablet core comprises Polyox® WSR 303 having a molecular weight of about 7,000,000 daltons.

In a preferred embodiment, the weight ratio of the inner layer with active ingredient to the coating layer is preferably within the range from 0.5 to 2 (w/w), more preferably from 0.5 to 1.5 (w/w).

In a preferred embodiment, the weight ratio of the inner layer with active ingredient to the mini tablet core is preferably within the range from 0.1 to 0.35 (w/w), more preferably from 0.1 to 0.2 (w/w).

Selection of the excipients and the sort of the layers in the formulation is the most important thing for releasing the active ingredient into the surrounding media in desired time. The water-soluble polymer is employed with this specific amount of the invention to control the swelling of the mini tablet core. After the coated paliperidone mini tablets meet with the media; passages, apertures, bores, holes or openings are occured on the coating layer to form a connection between the core and the surrounding media. In this present invention, the active ingredient is only situated in the inner layer. This special structuring of the present invention achieves to control the release of paliperidone from the tablet based on the desired drug-release profile.

The term "extended-release", as used herein, refers to a dosage form comprising a drug which is formulated in such a way so as to provide a longer duration of pharmacological response compared to an immediate-release dosage form comprising the same drug in the same amount. In particular, the term "extended-release", as used herein, refers to the release of paliperidone over a prolonged period of time, for example, over a period of 6 hours, 8 hours, 12 hours, 16 hours, or 24 hours.

In this present invention, the extended release pharmaceutical composition for oral administration extends the release of paliperidone or a pharmaceutically acceptable salt thereof over a period of at least 12 hours, preferably up to 24 hours.

According to this embodiment, in dissolution test, the time from the release of 5% to 15% of said paliperidone is at least 6 hours.

In another embodiment, in dissolution test, the pharmaceutical composition exhibits less than 100% release of said paliperidone at 24 hours.

In another embodiment, the pharmaceutical composition exhibits less than 15% release in 6 hours; 40-60% release in 12 hours; and 80-95% release in 18 hours.

For the present invention, the dissolution testing is carried out in 500 ml of [NaCl (0.2% w/w) in 0.0825N HCl] at pH 1.0 in a Type II apparatus at 50 rpm paddle speed.

The term "lag period" as used herein means the time period wherein at the most 5% of atypical antipsychotic such as paliperidone or its pharmaceutically acceptable salt is released from the composition.

Compositions based on one of the embodiments of the invention exhibits desired in vitro release of paliperidone or a pharmaceutically acceptable salt thereof with lag period.

In another embodiment, the composition exhibits desired in vitro release of paliperidone or a pharmaceutically acceptable salt thereof after the lag period of at least 2 hours.

Suitable osmotic agent is selected from magnesium chloride, magnesium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, and potassium dihydrogen phosphate; water-soluble salts of organic acids such as sodium acetate, potassium acetate, magnesium succinate, sodium benzoate, sodium citrate, and sodium ascorbate; carbohydrates such as mannitol, sorbitol, arabinose, ribose, xylose, glucose, fructose, mannose, galactose, sucrose, maltose, lactose, and raffinose; water-soluble amino acids such as glycine, leucine, alanine, and methionine; urea and urea derivatives; or mixtures thereof.

In a preferred embodiment, the osmotic agent is preferaly sodium chloride.

Suitable lubricant is selected from magnesium stearate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

In a preferred embodiment, the lubricant is preferaly magnesium stearate.

Suitable coating material is selected from polyvinylalcohol based films, polyethylene glycol, ethyl acrylate and methyl methacrylate copolymer dispersion, iron oxide yellow, polyvinyl alcohol, polyvinyl alcohol-polyethylene glycol copolymers, hydroxypropyl methyl cellulose, ethyl cellulose, ethylcellulose dispersions, polyvinylprolidone, polyvinylprolidone-vinyl acetate copolymer (PVP-VA), pigments, dyes, titanium dioxide, iron oxide, talc, iron oxide, triacetin , polymethylmetacrylate copolymers or mixtures thereof.

In a preferred embodiment, the coating material is preferaly polyvinylalcohol based films, polyethylene glycol, ethyl acrylate and methyl methacrylate copolymer dispersion or mixtures thereof.

In the process of preparation of the pharmaceutical formulation, the blends are granulated by conventional techniques known in the art such as wet granulation, dry granulation, extrusion-spheronization or hot melt extrusion. Wet granulation process involves the use of water or any other suitable granulating fluid. Dry granulation may involve the use of a roller compactor or any suitable technique.

The pharmaceutical formulation is prepared by combining the granule or blend compositions of the drug layers and the mini tablet core using appropriate conventional tooling.

The inner layer with active ingredient and coating layer is applied using conventional coating techniques well known in the art such as spray coating in a conventional coating pan or fluidized bed processor, dip coating, melt coating or compression coating.

In a preferred embodiment of the present invention, a spray coating technique is used.

An extended release pharmaceutical composition for oral administration, wherein the production process comprising the steps of:
- The core is pressed as mini tablet without active ingredient.
- The mini tablet is coated by a mixture with paliperidone or a pharmaceutically acceptable salt thereof.
- Finally, a coating layer is applied to the paliperidone mini tablets.
- The coated paliperidone mini tablets are brought into dosage forms which is a capsule.

### Example: Paliperidone mini tablets (1.5, 3, 6, 9, 12 mg paliperidone capsules)

**Mini tablet core:**

| Ingredients | Amount (mg) |
|---|---|
| Polyox* | 16 |
| Sodium chloride | 8.75 |
| Magnesium stearate | 0.25 |

**Inner layer comprising paliperidone:**

| Ingredients | Amount (mg) |
|---|---|
| Paliperidone | 0.75 |
| Opadry Clear** | 2.5 |
| Water | 23.85 |

**Coating layer:**

| Ingredients | Amount (mg) |
|---|---|
| Eudragit NE 30 D | 12.7 |
| Opadry II*** | 1 |
| PEG 400 | 0.11 |
| PEG 6000 | 0.11 |
| Water | 4.8 |

| | |
|---|---|
| *Polyox: polyethylene oxide ** Opadry Clear: hydroxypropyl methyl cellulose, polyethylene glycol 400, polyethylene glycol 6000 *** Opadry II: polyvinyl alcohol partially hydrolysed, titanium dioxide, polyethylene glycol 3000, talc | |

### Production process:

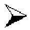 Polyox, sodium chloride, magnesium stearate are mixed and compressed as mini tablets.
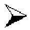 Opadry Clear is dispersed in water. The paliperidone is added to this mixture and mixed. The mini tablets are coated with this inner layer mixture.
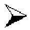 PEG 6000 is dissolved in water. Opadry II is added and mixed. Then Eudragit NE 30 D and PEG 400 are added respectively and mixed homogeneously. The mini tablets which is coated by the mixture with paliperidone are coated again with ths mixture.
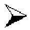 The coated paliperidone mini tablets are filled into capsules.
   - For dosage form comprising 1.5 mg paliperidone, 2 mini tablets are used for every capsule.
   - For dosage form comprising 3 mg paliperidone, 4 mini tablets are used for every capsule.
   - For dosage form comprising 6 mg paliperidone, 8 mini tablets are used for every capsule.
   - For dosage form comprising 9 mg paliperidone, 12 mini tablets are used for every capsule.
   - For dosage form comprising 12 mg paliperidone, 18 mini tablets are used for every capsule.

## Claims

1. An extended release pharmaceutical composition for oral administration comprising;
• a mini tablet core comprising one or more water-soluble polymers,
• an inner layer comprising paliperidone or a pharmaceutically acceptable salt thereof,
• a coating layer.

2. The pharmaceutical composition according to claim 1, wherein the dosage form is in the form of a capsule.

3. The pharmaceutical composition according to claim 2, wherein paliperidone is present as paliperidone base is in an amount between 1.5 - 12 mg.

4. The pharmaceutical composition according to claim 3, wherein paliperidone is preferably in an amount of 1.5 mg, 3 mg, 6 mg, 9 mg and 12 mg.

5. The pharmaceutical composition according to claim 1, wherein paliperidone in an amount of 1.5% to 2.5% of total weight of the pharmaceutical composition, more preferably 1.7% to 2.3% of total weight of the pharmaceutical composition.

6. The pharmaceutical composition according to claim 1, wherein the inner layer comprises paliperidone in an amount of 5% to 30%, more preferably 8% to 25% of total weight of the inner layer.

7. The pharmaceutical composition according to any of the preceeding claims, comprising at least one pharmaceutically acceptable excipient which is selected from water-soluble polymers, osmotic agents, lubricants, coating materials or mixtures thereof.

8. The pharmaceutical composition according to claim 7, wherein the water-soluble polymer is selected from hydroxypropyl cellulose (HPC), hydroxypropyl methylcellulose (HPMC), hydroxypropyl methylcellulose E3, E5 and E15 and K-3, hydroxyethylcellulose and derivatives, ethylcellulose and ethyl hydroxyethylcellulose, carboxymethylcellulose (CMC), carboxymethylcellulose cekol 30, methyl cellulose (MC), methyl cellulose A-3, A6 and A-15; polyvinyl alcohol (PVA), polyethylene glycol (PEG), polyethylene oxide (PEO), poly(ethylene oxide-b-propylene oxide), polyoxyethylene (POE), poly vinyl pyrrolidone (PVP), polyethylenimine (PEI), poly(N-vinylpyrrolidone/vinyl acetate), polyvinylpyrollidone PVP K-90, polyacrylic acid and copolymers, poly(vinylamine) hydrochloride, poly(acrylic acid sodium salt), poly(methacrylic acid), poly(methylacrylic acid sodium salt), poly(ethylene/acrylic acid), poly(2-hydroxyethyl methacrylate/methacrylic acid), poly(2-hydroxypropyl methacrylate), polyacrylamides (PAM), poly(acrylamide/acrylic acid), polymethacrylamide, poly(N-isopropylacrylamide) (PNIPAM), poly(styrenesulfonic acid) sodium salt, poly(2-oxazoline), poly(2-ethyl-2-oxazoline), poly(3-chloro-2-hydroxypropyl-2-methacryloxyethyldimethylammonium chloride), poly(2-vinyl-1-methylpyridinium bromide), poly(2-vinylpyridine), polyamines, poly(2-vinylpyridine N-oxide), poly(N-vinylpyrrolidone/2-dimethylaminoethyl methacrylate) dimethyl sulfate quaternary, poly(4-vinylpyridine N-oxide), poly(4-vinylpyridine), poly(styrenesulfonic acid/maleic acid) sodium salt, poly(N-vinyl acetamide), poly(N-vinyl acetamide-co-sodium acrylate), poly(vinylsulfonic acid) sodium salt, polyelectrolytes; agar, alginates, casein and caseinates, carrageenan, chitosan, cucurbit[n]uril hydrate, curdlan, dextran, gelatin, gellan gum, guar gum and derivatives, gum carrageenan, gum arabic and other tree and shrub exudates, locust bean gum, maltodextrins, methocel, pectin, pullulan, resin, poly(ethylene oxide), poly(ethylene oxide) resin, sodium alginate, starch and starch derivatives, xanthan gum or mixture thereof.

9. The pharmaceutical composition according to claim 1, wherein water-soluble polymer in an amount of more than about 50% w/w, and in particular more than about 70% w/w of the total weight of the mini tablet core.

10. The pharmaceutical composition according to claim 1, wherein water-soluble polymer in an amount of more than about 40% w/w, and in particular more than about 55% w/w of the total weight of the pharmaceutical composition.

11. The pharmaceutical composition according to claim 1, wherein the weight ratio of water-soluble polymer to paliperidone is 15 to 30.

12. The pharmaceutical composition according to sny claims 8 to 11, wherein the water-soluble polymer is preferaly polyethylene oxide.

13. The pharmaceutical composition according to claim 7, wherein the osmotic agent is selected magnesium chloride, magnesium sulfate, lithium chloride, sodium chloride, potassium chloride, lithium hydrogen phosphate, sodium hydrogen phosphate, potassium hydrogen phosphate, lithium dihydrogen phosphate, sodium dihydrogen phosphate, and potassium dihydrogen phosphate; water-soluble salts of organic acids such as sodium acetate, potassium acetate, magnesium succinate, sodium benzoate, sodium citrate, and sodium ascorbate; carbohydrates such as mannitol, sorbitol, arabinose, ribose, xylose, glucose, fructose, mannose, galactose, sucrose, maltose, lactose, and raffinose; water-soluble amino acids such as glycine, leucine, alanine, and methionine; urea and urea derivatives; or mixtures thereof.

14. The pharmaceutical composition according to claim 7, wherein the lubricant is selected from magnesium stearate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

15. The pharmaceutical composition according to claim 7, wherein the coating material is selected from polyvinylalcohol based films, polyethylene glycol, ethyl acrylate and methyl methacrylate copolymer dispersion, iron oxide yellow, polyvinyl alcohol, polyvinyl alcohol-polyethylene glycol copolymers, hydroxypropyl methyl cellulose, ethyl cellulose, ethylcellulose dispersions, polyvinylprolidone, polyvinylprolidone-vinyl acetate copolymer (PVP-VA), pigments, dyes, titanium dioxide, iron oxide, talc, iron oxide, triacetin , polymethylmetacrylate copolymers or mixtures thereof.

16. The pharmaceutical composition according to any of the preceeding claims, wherein the production process comprising the steps of:
• Pressing the core as mini tablet without active ingredient.
• Coating the mini tablet by a mixture with paliperidone or a pharmaceutically acceptable salt thereof.
• Finally, applying a coating layer to the paliperidone mini tablets.
• Bringing the coated paliperidone mini tablets into dosage forms which is a capsule.
